# EUROPÄISCHE PATENTANMELDUNG

(11) **EP 2 505 134 A1**
(43) Veröffentlichungstag der Anmeldung: **03.10.2012**
(21) Anmeldenummer: 11190833.1
(22) Anmeldetag: 25.11.2011
(51) Int. Cl.: A61B 5/04, A61B 5/02, A61M 1/14, A61N 1/00

(54) **Vorrichtung zur Detektion und/oder Beeinflussung der Gerinnung von Blutsystemen, umfassend zwei Elektroden, und entsprechendes Verfahren**

(30) Priorität: 29.03.2011 DE 102011006349
(71) Anmelder: Fraunhofer-Gesellschaft zur Förderung der angewandten Forschung e.V., 80686 München (DE)
(72) Erfinder: Eyerer, Peter, 76228 Karlsruhe (DE); Bender, Angelika Dorothea, 70565 Stuttgart (DE)
(74) Vertreter: Haggenmüller, Christian

(57) **Zusammenfassung**

Die vorliegende Erfindung betrifft eine zumindest teilweise in einem Blutsystem anordenbare Vorrichtung zur Detektion, Vermessung und/oder Beeinflussung der Gerinnung von Blutsystemen, umfassend eine erste Elektrode, eine zweite Elektrode,
wobei die Elektroden zumindest teilweise in das Blutsystem eintauchbar sind und mit einem Messgerät zur Erfassung mindestens einer elektrischen Messgröße und optional mit einer Spannungs- und/oder Stromquelle elektrisch leitend verbindbar sind. Die gemessenen elektrischen Werte ermöglichen es, einen beginnenden Gerinnungsvorgang zu erkennen. Durch Aktivieren der Strom- bzw. Spannungsquelle ist es möglich, mit hilfe des äußeren elektrischen Gegenfeldes die Blutgerinnung zu beeinflussen, insbesondere zu vermindern.

## Beschreibung

Im Bereich der Medizintechnik wäre es von Interesse, die Ausbildung von Blutgerinnseln möglichst frühzeitig zu detektieren und gegebenenfalls durch entsprechende Gegenmaßnahmen zu beeinflussen, insbesondere zu vermindern oder zu unterbinden.

Dies wäre z.B. für folgende Anwendungen bedeutsam:
- Detektion von Blutgerinnseln, insbesondere im Umfeld von metallischen Implantaten,
- Überwachung und Überprüfung von Vorrichtungen zur Lagerung und/oder zum Transport von Blut, wie Dialysegeräte zur Umwälzung von Blut,
- Untersuchung von medizintechnischen Produkten im Hinblick auf Gerinnung,
- Verminderung bis Unterdrückung der Bildung von Blutgerinnseln, insbesondere in technischen Geräten.

Patienten mit medizinischen Implantaten wie z.B. einem Stent wird häufig Heparin als Substanz zur Blutgerinnungshemmung verabreicht. Dabei kann es aber zu Unverträglichkeiten oder auch zu einer "überschießenden" Blutungsneigung kommen. Von Interesse wäre daher ein Stent, der so ausgestaltet ist, dass er die Ausbildung eines Blutgerinnsel frühzeitig detektiert und gegebenenfalls sogar unterbindet, um so eine reduzierte Anwendung von Heparin zu ermöglichen.

Auch in medizinischen Geräten zur Lagerung oder zum Transport von Blut wie z.B. Dialysegeräten und Blutinfusionsgeräten ist eine Blutgerinnung bzw. Gerinnselbildung unerwünscht und sollte möglichst zeitnah zu ihrer Ausbildung detektiert werden, um geeignete Gegenmaßnahmen einleiten zu können.

Unter Berücksichtigung der obigen Ausführungen ist eine Aufgabe der vorliegenden Erfindung darin zu sehen, ein medizinisches Implantat, insbesondere einen Stent, sowie ein medizinisches Gerät zur Lagerung oder zum Transport von Blut (wie z.B. Dialysegeräte und Blutinfusionsgeräte) bereit zu stellen, die jeweils die Ausbildung eines Blutgerinnsels frühzeitig detektieren und gegebenenfalls sogar unterbinden können.

Eine weitere Aufgabe der vorliegenden Erfindung besteht in der Bereitstellung eines Verfahrens, mit dem möglichst effizient die Ausbildung eines Blutgerinnsels detektiert und beeinflusst, gegebenenfalls sogar unterbunden werden kann, wobei dieses Verfahren aber möglichst keinen negativen Einfluss auf die Blutqualität haben sollte (also möglichst schonend und "zerstörungsfrei" arbeiten sollte).

Wie nachfolgend noch eingehend erläutert wird, wurde im Rahmen der vorliegenden Erfindung festgestellt, dass diese Aufgabenstellungen unter Ausnutzung von besonderen elektrischen Effekten gelöst werden können. Zum besseren Verständnis der Erfindung werden die Grundlagen dieser Effekte kurz erläutert.

Im Bereich der Polymere ist bekannt, dass bei der Aushärtung von Epixdharz-Systemen ein elektrischer Effekt auftritt, welcher zuvor als Costa-Ribeiro-Effekt (thermodielektrischer Effekt) für Naphthalin bekannt geworden ist und dem für

Wasser gefundenen Workman-Reynolds-Effekt ähnelt. Siehe z.B. P. Eyerer, "Eine zerstörungsfreie elektrische Prüfmethode zur Überwachung von Aushärtevorgängen an Duromeren", Dissertation an der Universität Stuttgart, 1972. Die Grundlage beider Effekte ist eine Ladungstrennung an einer wandernden Phasenfront zwischen zwei Zustandsformen. Verläuft diese Phasenfront parallel und eben zu zwei Elektroden, so kann im äußeren Stromkreis je nach Messanordnung eine Spannung oder ein Strom gemessen werden. Das Auftreten dieses thermodielektrischen Effekts wurde dazu genutzt, Vernetzungsvorgänge an Duromeren zu untersuchen.

Dielektrische Messungen haben in der Kunststoffverarbeitung Anwendung als zerstörungsfreie, schnell durchführbare Prüfverfahren gefunden. Hierbei wird der Aushärtungsverlauf polymerer Systeme, wie Elastomere und Duromere (polymere Harzsysteme), oder das Schmelzen und Erstarren thermoplastischer Kunststoffe erfasst.

Als Messanordnung kommt beispielsweise eine Platte-Platte-Kondensatoranordnung in Frage, die sich in der Regel relativ einfach in die Kavität eines Werkzeuges einbauen lässt und die über das gesamte, zwischen den Elektroden befindliche Volumen mittelt. Im Labor lässt sich eine solche Messzelle ebenfalls gut realisieren. An die heizbare Messzelle (Plattenkondensator) sind eine Messbrücke und ein Rechner angeschlossen. Die Probe befindet sich zwischen den beiden Elektroden und kann bei Bedarf auf über 200°C aufgeheizt werden.

Die zu untersuchenden Prüfsubstanzen werden zwischen Elektroden, die sowohl symmetrisch als auch asymmetrisch beheizt werden können, ausgehärtet. Als charakteristische Reaktionspunkte werden Vernetzungsbeginn, Gelpunkt und Vernetzungsende erfasst, wobei die Untersuchung des Vernetzungspotentials eine zerstörungsfreie Prüfmethode für Vernetzungsvorgänge darstellt. Bei einem Epoxidharz-Amid-System treten zwei Teilreaktionen, eine Polymerisation und eine Polyaddition auf, was durch ultraspektrometrische Untersuchungen bestätigt werden kann.

Als Gesetzmäßigkeiten des thermodielektrischen Effekts sind Proportionalitäten zwischen dem bei isothermem Schmelzen oder Erstarren fließenden Strom und der Phasenfortbewegungsgeschwindigkeit sowie zwischen der in einem Zweiphasensystem getrennten elektrischen Ladungsmenge und der Massenänderung einer Phase postuliert und für verschiedene Systeme, wie Naphthalin oder Camauba-Wachs, experimentell nachgewiesen worden. Das Grundprinzip einer Ladungstrennung an einer wandernden Phasenfront ist für Naphthalin und ein EisWasser-Gemisch gleich und konnte auch für Kunststoffsysteme, beispielsweise Epoxidharzsysteme und (ungesättigtes) Polyesterharz, Polyethylen, Polyamid, gezeigt werden.

Die Veränderungen der dielektrischen Messgrößen während der Aushärtung von polymeren Systemen sind bei konstanter Temperatur ausschließlich auf Veränderungen des Aushärtungszustandes zurückzuführen, weil die Beweglichkeit der im Harz vorhandenen Dipole und die der Ladungsträger abnehmen. Währen sich Dipole bei niedrigen Messfrequenzen im flüssigen, unvernetzten Harz noch vollständig im elektrischen Feld ausrichten können, d.h., relaxieren, gelingt dies mit höherer Frequenz und zunehmender Aushärtung des Harzes immer schlechter. Bei der Beschreibung der temperaturabhängigen Verschiebung von nichtreaktiven Systemen geht man davon aus, dass durch eine Temperaturerhöhung die Beweglichkeit von Ladungsträgern und Dipolen erhöht wird und damit zu einer Verschiebung der Frequenzspektren zu höheren Frequenzen führt.

Bei einer festen Frequenz wird die Beweglichkeit der vorhandenen Ladungsträger als Funktion der Aushärtezeit errechnet, um damit die Viskositätsänderung von Harzen während des Aushärteprozesses zu beschreiben. Um das dielektrische Messverfahren beispielsweise in der Produktion zur Qualitätssicherung einzusetzen, wurden Modelle entwickelt, den Reaktionsverlauf des Aushärteprozesses anhand der dielektrischen Messdaten zu beschreiben. Neben einem Zeit-Temperatur-Verschiebungsgesetz konnte die Gültigkeit eines Zeit-Vernetzungszeit-Verschiebungsgesetz für verschiedene Harzsysteme experimentell nachgewiesen werden.

Es wurde nun überraschend festgestellt, dass sich diese elektrischen Effekte auch bei der Lösung der oben beschriebenen Aufgabenstellungen erfolgreich anwenden lässt.

Gemäß eines ersten Aspekts der vorliegenden Erfindung wird eine zumindest teilweise in einem Blutsystem anordenbare Vorrichtung zur Detektion, Vermessung und/oder Beeinflussung der Gerinnung von Blutsystemen bereitgestellt, umfassend:
- eine erste Elektrode,
- eine zweite Elektrode,
wobei die Elektroden zumindest teilweise in das Blutsystem eintauchbar sind und mit einem Messgerät zur Erfassung mindestens einer elektrischen Messgröße und optional mit einer Spannungs- und/oder Stromquelle elektrisch leitend verbindbar sind.

Bevorzugt sind die Elektroden aus einem Metall, bevorzugt einem Edelmetall, besonders bevorzugt Gold, gefertigt.

Im Rahmen der vorliegenden Erfindung kann die räumliche Form bzw. Ausgestaltung der Elektroden über einen breiten Bereich variiert werden. Es kann in diesem Zusammenhang auf herkömmliche, dem Fachmann bekannte Elektrodenformen verwiesen werden. Beispielsweise können die Elektroden in herkömmlicher Weise als Plattenelektroden, zylinderförmige Elektroden, Drahtelektroden oder auch Stiftelektroden ausgebildet sein. Die Platten- bzw. zylinderförmigen Elektroden können auch aus einem Material in Netzform (z.B. Edelmetallnetz, bevorzugt Goldnetz) gebildet sein. In einer bevorzugten Ausführungsform umfasst die erste bzw. zweite Elektrode oder alternativ umfassen beide Elektroden jeweils einen Rahmen, in dem ein Edelmetallnetz (z.B. Goldnetz) angebracht bzw. eingespannt ist.

Somit können erste und zweite Elektrode und Blutplasma je nach verwendeter Elektrodenform z.B. als eine Plattenkondensator- oder auch eine Zylinderkondensatoranordnung vorliegen.

Zur besseren Fixierung und definierten räumlichen Anordnung der Elektroden können Trägermaterialien verwendet werden. Dafür kommen herkömmliche, dem Fachmann bekannte Materialien in Frage. Als bevorzugtes Trägermaterial wird z.B. ein thermoplastischer Kunststoff wie PVC oder Poly(meth)acrylate verwendet.

Bevorzugt sind die Elektroden als Plattenelektroden, zylinderförmige Elektroden, Drahtelektroden und/oder Stiftelektroden ausgebildet.

Als geeignete elektrische Messgrößen können z.B. Stromstärke und Spannung genannt werden. Messgeräte zur (gesonderten oder gleichzeitigen) Bestimmung dieser Größen sind dem Fachmann grundsätzlich bekannt.

Bei dem Blutsystem kann es sich z.B. um Blut oder Blutplasma handeln.

Bei der Vorrichtung kann es sich um eine solche handeln, die außerhalb des menschlichen Körpers zur Detektion, Vermessung und/oder Beeinflussung der Gerinnung von Blutsystemen verwendet wird. Alternativ kann es sich z.B. um eine implantierbare Vorrichtung handeln.

Gemäß eines weiteren Aspekts der vorliegenden Erfindung wird ein implantierbares System, insbesondere Stentanordnung, mit der oben beschriebenen Vorrichtung bereitgestellt.

Im Rahmen der vorliegenden Erfindung wird der Begriff "Stent" in seiner üblichen, dem Fachmann geläufigen Weise verwendet und bezieht sich daher auf eine Vorrichtung, die als medizinisches Implantat in Hohlorgane wie z.B. Blutgefäße eingebracht wird, um diese offen zu halten.

Bevorzugt ist in dem System die erste Elektrode als ein erster rohrförmiger Körper mit einem Innendurchmesser D1 und einer Wandung, die ein Innenvolumen V₁ umschließt, ausgebildet.

Hinsichtlich eines geeigneten strukturellen Aufbaus des ersten rohrförmigen Körpers kann auf die Struktur üblicher, dem Fachmann bekannter Stents verwiesen werden. So können die Wandungen der rohrförmigen Körper z.B. aus einem gitter- bzw. netzförmigem Material gefertigt sein. Alternativ ist es auch möglich, dass die Wandungen der rohrförmigen Körper zumindest abschnittsweise ein flächiges, flüssigkeitsundurchlässiges Material aufweisen.

Hinsichtlich der Auswahl geeigneter Innendurchmesser D1 und Wanddicken des ersten rohrförmigen Körpers kann auf die Abmessungen üblicher Stents verwiesen werden. Geeignete Werte hängen unter anderem auch von den Abmessungen des Hohlorgans, in das der Stent eingebracht wird, ab.

Bevorzugt ist in dem System die zweite Elektrode als ein zweiter rohrförmiger Körper mit einem Außendurchmesser D2, wobei D2<D1, und/oder als eine stift- oder drahtförmige Komponente ausgebildet.

Hinsichtlich eines geeigneten strukturellen Aufbaus des zweiten rohrförmigen Körpers kann auf die obigen Ausführungen zu dem ersten rohrförmigen Körper verwiesen werden.

Wird ein zweiter rohrförmiger Körper verwendet, so weist dieser einen Außendurchmesser D2 auf, der geringer ist als der Innendurchmesser D1 des ersten rohrförmigen Körpers. Dadurch wird es ermöglicht, dass der zweite rohrförmige Körper in dem von der Wandung des ersten rohrförmigen Körper umschlossenen Innenvolumen V₁ angebracht und somit zumindest teilweise von der Wandung des ersten rohrförmigen Körpers umschlossen ist.

Die Wandungen des ersten und zweiten rohrförmigen Körpers sollten bevorzugt ausreichend voneinander beabstandet sein, so dass nach einer Implantation in ein Blutgefäß der Blutfluss nicht beeinträchtigt würde. Z.B. ist D2 ≤ 0.8 x D1.

Geeignete Innendurchmesser und Wanddicken des zweiten rohrförmigen Körpers werden bevorzugt so ausgewählt, dass nach erfolgter Implantation in ein Blutgefäß der Blutfluss nicht beeinträchtigt würde.

Alternativ oder ergänzend zu dem zweiten rohrförmigen Körper kann das implantierbare System eine stift- oder drahtförmige Komponente aufweisen. Diese kann z.B. in Form eines Drahtes ausgebildet sein. Die Wahl geeigneter Durchmesser der stift- oder drahtförmigen Komponente hängt von den Abmessungen des ersten rohrförmigen Körpers ab.

Bevorzugt ist/sind der zweite rohrförmige Körper und/oder die stift- oder drahtförmige Komponente in dem Innenvolumen V₁ angebracht und somit zumindest teilweise von der Wandung des ersten rohrförmigen Körpers umschlossen.

Bevorzugt sind der zweite rohrförmige Körper und/oder die stift- oder drahtförmige Komponente und der erste rohrförmige Körper koaxial zueinander angeordnet sind.

Wie nachfolgend noch eingehender erläutert wird, ermöglicht das erfindungsgemäße implantierbare System nach seiner Implantation die Detektion von Blutgerinnseln in einem schonenden Verfahren, das auch ohne Anlegen eines äußeren elektrischen Feldes einen beginnenden Gerinnungsvorgang erkennen und gegebenenfalls beeinflussen kann.

Da die Verwendung der erfindungsgemäßen Vorrichtung bzw. des erfindungsgemäßen Systems die Detektion eines Blutgerinnsels auch ohne Anlegen eines externen elektrischen Feldes an die Elektroden ermöglicht, ist es im Rahmen der vorliegenden Erfindung möglich, dass die Vorrichtung lediglich ein Messgerät zur Erfassung mindestens einer elektrischen Messgröße (z.B. Stromstärke oder Spannung), nicht jedoch auch eine Strom- bzw. Spannungsquelle zum Anlegen eines externen elektrischen Feldes aufweist.

Umfasst die Vorrichtung bzw. das System zusätzlich auch eine Strom- bzw. Spannungsquelle, so kann mit dieser ein äußeres elektrisches Gegenfeld generiert werden, das gegenüber dem durch den thermodielektrischen Effekt erzeugten Feld entgegengesetzt ausgerichtet, jedoch bevorzugt von ähnlicher Feldstärke ist. Dadurch kann die Gerinnung beeinflusst, insbesondere vermindert oder sogar unterdrückt werden.

In einer bevorzugten Ausführungsform kann die Vorrichtung bzw. das System weiterhin zusätzliche Messgeräte zur Messung von physikalischen Parametern des Blutsystems aufweisen. Dabei kann es sich z.B. um optische und/oder rheologische Messgeräte handeln, mit denen die durch die Gerinnung bewirkte Zustandsänderung des Blutes detektiert werden kann. Mit diesen zusätzlichen Messgeräten kann die Empfindlichkeit der Vorrichtung hinsichtlich der Gerinnungsdetektion erhöht werden. Auch können diese zusätzlichen Messgeräte dazu dienen, einen geeigneten oberen kritischen Grenzwert hinsichtlich der elektrischen Messgröße festzulegen. Solche Messgeräte, insbesondere optische oder rheologische Messgeräte, sind dem Fachmann grundsätzlich bekannt.

Gemäß eines weiteren Aspekts betrifft die vorliegende Erfindung eine Anordnung mit der oben beschriebenen Vorrichtung oder dem oben beschriebenen System und einem Computer, der programmtechnisch so eingerichtet ist, dass bei dem Überschreiten eines kritischen Messwertes der elektrischen Messgröße ein äußeres elektrisches Gegenfeld durch die Strom- bzw. Spannungsquelle generiert wird und bevorzugt beim Unterschreiten des kritischen Grenzwertes wieder deaktiviert wird.

Gemäß eines weiteren Aspekts betrifft die vorliegende Erfindung ein Gerät zur Blutbehandlung und/oder Blutaufbewahrung außerhalb des menschlichen Körpers, enthaltend die beschriebene Vorrichtung oder Anordnung, wobei es sich bei dem Gerät bevorzugt um ein Dialysegerät oder ein Blutinfusionsgerät handelt.

Gemäß eines weiteren Aspekts betrifft die vorliegende Erfindung ein Verfahren zur Detektion, Vermessung und/oder Beeinflussung der Gerinnung von Blutsystemen außerhalb des menschlichen Körpers, umfassend:
- die Bereitstellung einer Vorrichtung, bevorzugt der oben beschriebenen Vorrichtung, die zumindest eine erste Elektrode und eine zweite Elektrode umfasst, wobei die Elektroden mit einem Messgerät zur Erfassung mindestens einer elektrischen Messgröße und optional mit einer Spannungs-und/oder Stromquelle elektrisch leitend verbunden sind,
- in Kontakt bringen der Elektroden mit einem Blutsystem, wie Blutplasma oder Blut, wobei die Elektroden zumindest teilweise in das Blutsystem eingetaucht sind und kein äußeres elektrisches Feld an die Elektroden angelegt wird,
- Messung der zumindest einen elektrischen Messgröße.

Bevorzugt wird das Verfahrens unter Verwendung der oben beschriebenen Vorrichtung und/oder der oben beschriebenen Anordnung und/oder dem oben beschriebenen Gerät durchgeführt.

Hinsichtlich der bevorzugten Eigenschaften der Elektroden und des Messgeräts zur Erfassung mindestens einer elektrischen Messgröße kann auf die obigen Ausführungen verwiesen werden.

Wie oben erwähnt, wird an die in das Blutplasma eingetauchte Elektrodenanordnung zunächst kein äußeres elektrisches Feld angelegt, d.h. die Spannungs- bzw. Stromquelle, sofern vorhanden, ist noch nicht eingeschaltet bzw. aktiviert.

In einer bevorzugten Ausführungsform des Verfahrens wird ein kritischer Grenzwert der elektrischen Messgröße festgelegt, werden die gemessenen Werte der elektrischen Messgröße mit dem festgelegten kritischen Grenzwert abgeglichen, und erfolgt durch die Spannungs- oder Stromquelle beim Überschreiten des kritischen Grenzwertes die Generierung eines äußeren elektrischen Gegenfeldes, das gegenüber dem durch die elektrische Messgröße generierten elektrischen Feld entgegengesetzt ausgerichtet ist.

Somit wird bevorzugt beim Überschreiten des kritischen Grenzwertes die mit den Elektroden verbundene Strom- bzw. Spannungsquelle eingeschaltet bzw. aktiviert und erzeugt ein entsprechendes Gegenfeld.

Wie bereits oben erläutert, führt der bei der Blutgerinnung auftretende thermodielektrische Effekt dazu, dass in der Elektrodenanordnung auch ohne Anlegen eines äußeren Feldes (d.h. ohne Aktivierung der Strom- bzw. Spannungsquelle) eine elektrische Messgröße mit einem bestimmbaren Messwert induziert wird. Diese elektrische Messgröße wie z.B. Spannung oder Stromstärke erzeugt wiederum ein elektrisches Feld (d.h. letztendlich ein durch den thermodielektrischen Effekt generiertes elektrisches Feld).

Bevorzugt entspricht die Stärke des durch die Spannungs- bzw. Stromquelle generierten äußeren elektrischen Gegenfeldes etwa der Stärke des durch die elektrische Messgröße (und damit durch den thermodielektrischen Effekt) generierten elektrischen Feldes.

Durch das Anlegen eines entsprechenden Gegenfeldes ist es möglich, die Blutgerinnung zu beeinflussen, z.B. zu unterdrücken oder deren Ausmaß zumindest zu reduzieren.

Die Festlegung eines geeigneten kritischen Grenzwerts der elektrischen Messgröße kann in Abhängigkeit davon erfolgen, wie empfindlich das Verfahren auf eine beginnende Gerinnung reagieren soll.

Wie bereits oben ausgeführt, kann es vorteilhaft sein, zusätzliche Messgeräte zur Messung von physikalischen Parametern des Blutplasmas einzusetzen. Dabei kann es sich z.B. um optische und/oder rheologische Messgeräte handeln, mit denen die durch die Gerinnung bewirkte Zustandsänderung des Blutes detektiert werden kann. Mit diesen zusätzlichen Messgeräten kann die Empfindlichkeit der Vorrichtung hinsichtlich der Gerinnungsdetektion erhöht werden. Auch können diese zusätzlichen Messgeräte dazu dienen, einen geeigneten oberen kritischen Grenzwert hinsichtlich der elektrischen Messgröße festzulegen.

Bevorzugt wird beim Unterschreiten des kritischen Grenzwertes das äußere elektrische Gegenfeld wieder deaktiviert. Somit wird sichergestellt, dass das äußere Feld nur über einen begrenzten Zeitraum vorliegt und somit ein schonendes Verfahren zur Detektion und gegebenenfalls zur Beeinflussung der Blutgerinnung ermöglicht.

In einer bevorzugten Ausführungsform werden die ermittelten Messwerte der elektrischen Messgröße an einen Computer übermittelt, der programmtechnisch so eingerichtet ist, dass bei dem Überschreiten des kritischen Messwertes (z.B. kritischer Spannungswert) das äußere elektrische Gegenfeld durch die Strom- bzw. Spannungsquelle generiert und beim Unterschreiten des kritischen Grenzwertes bevorzugt wieder deaktiviert wird.

Weiterhin betrifft die vorliegende Erfindung die oben beschriebene Vorrichtung bzw. das oben beschriebene implantierbare System zur Verwendung bei der Detektion, Vermessung und/oder Beeinflussung (insbesondere Reduzierung oder Unterdrückung) der Gerinnung von Blutsystemen.

Nachfolgend wird die Erfindung unter Bezugnahme auf beispielhafte Ausführungsformen eingehender beschrieben.

### Beispiele

Zum Nachweis der Detektionsmethode sind u.a. die nachfolgend dargestellten Versuche durchgeführt worden. Es haben sich reproduzierbare elektrische Effekte gezeigt, die mit der gut beobachtbaren Wanderung einer Gerinnungsfront bei transparentem, inhibiertem Blutplasma, das mit Calciumchlorid aktiviert werden kann, korrelieren.

Es ist eine Messeinrichtung konzipiert und realisiert worden, durch die Folgendes erreicht wird: Eine hohe Genauigkeit bei der optischen Vermessung; eine bestmögliche Vermeidung von Störungen durch äußere Lichtquellen, wie Tageslichtschwankungen; eine unterbrechungslose Vermessung des gesamten Gerinnungsverlaufs über mindestens 24 Stunden; die Erfassung von für eine weitere Auswertung zugänglichen Messdaten mit gleichem Zeitstempel.

Die konkreten Arbeitsschritte zur Vermessung einer Gerinnungsfront in Blutplasmasystemen können zum Beispiel umfassen: (1) Aufbau einer Messvorrichtung mit Küvettenhalterung, Kamera, Objektiv, elektrischer Messeinrichtung(en), Beleuchtung und Verdunklungseinrichtug; (2) Bereitstellung einer gleichzeigen Ansteuerung von Kamera und elektrischer Messeinrichtung(en), wie eines Elektrometers, mit definiertem Zeitfenster und Zeitstempel; (3) Entwicklung und Implementierung eines Programms zur Auswertung der Bilder des in Küvetten aufgenommenen Blutplasmasystems; Bestimmung von horizontaler Lage und Dicke der Gerinnungsfront bzw. -schicht ("Füllstandsmessung"); (4) Anfertigung und Anpassung elektrischer Messeinrichtung(en) an unterschiedliche Messanforderungen, wie Stromverlaufsmessung; (5) Anfertigung einer zur Kameraaufnahme zeitsynchronen Messwerterfassung zur Vermessung begleitender Effekte; (6) Funktionsnachweis der Apparatur zur automatisierten Vermessung des Gerinnungsverlaufs in Blutplasma; (7) automatisierte gleichzeitige optische und elektrische Vermessung des Gerinnungsverlaufs in Blutplasmasystemen.

Ein exemplarischer Versuchsaufbau umfasst folgende Komponenten: Optische Schiene: Länge 0,75 m; auf Schiene verschieb- und arretierbare Reiter; Farbkamera TM-6740GE der Firma Pulnix: 640 x 480 Pixel, Gigabit-Ethernet-Schnittstelle; Objektiv mit Zwischenring(en) der Firma Pentax; Blaues LED-Durchlicht der Firma Stemmer Imaging mit Netzgerät; Küvettenhalterung(en) zur Aufnahme einer Küvette; Verdunklungskiste aus Blech zur Umhausung der vorgenannten Komponenten; in Verdunklungskiste integrierter Lüfter; Elektrodenanordnung; Elektrometer 6517A der Firma Keithley: Eingangswiderstand 200TOhm, eingebaute Spannungsquelle mit 1kV, Strommessung bis <100aA, IEEE 488-Schnittstelle; Computer der Firma Dell, OptiPlex^{®} 740 Enhanced mit AMD Athlon(tm) 64X2 Dual Core Processor 5800 (3Ghz); RAM 1,982 MB; Windows^{®} XP Pro SP3; Master-Slave-Steckdosenleiste.

Über die Anordnung und Einstellung der Kamera zur Küvette bzw. Küvettenhalterung (Arbeitsabstand) sowie des Objektivs wird ein ortsfestes Bildfenster zur Beobachtung des Verlaufs der Gerinnungsfront festgelegt. Das Bildfenster wird derart eingestellt, dass es die senkrecht gehaltene Küvette nahezu vollständig beinhaltet. Weiter werden das Bildfenster und die Beleuchtung derart gewählt, dass ein Kontrast zwischen einem oberen Bereich mit geronnenem Blutplasma und einem unteren Bereich mit (noch) nicht geronnenem Blutplasma sichtbar vermessen werden kann.

Über das auf dem Computer installierte Programm zur Erfassung und Auswertung von Messdaten werden die Kamera und das Elektrometer zeitsynchronisiert zur Erfassung eines Bildes und zumindest eines Messwertes angesteuert. Aus den Bilddaten wird die Lage der Gerinnungsfront zwischen oberem und unterem Bereich zeit- und ortsaufgelöst bestimmt. Bei der optischen Vermessung gemäß Arbeitsschritt 3, genauer zur Bildverarbeitung, werden entsprechende Softwaremodule eingesetzt. Die am Versuchsaufbau implementierte Messtechnik erlaubt eine Korrelation mit den weiteren zeitsynchronisiert erfassten Messwerten elektrischer Messungen.

Es werden Einwegküvetten aus Kunststoff, genauer aus Polystyrol, eingesetzt. Hieraus ergibt sich der Vorteil, dass das Reinigen der Küvetten entfällt und als mögliche Fehlerquelle bei den Messungen ausscheidet. Die handelsüblichen Küvetten weisen ein Füllvermögen von 4 ml (sogenannte Makro-Küvetten) und zwei optisch klare Seiten auf. Kunststoffe sind in medizintechnischen Anwendungen, wie in zur Umwälzung von Blut, beispielsweise bei der Dialyse oder Bluttransfusion, eingesetzten Geräten weit verbreitet.

An die Elektrodenanordnungen sind bevorzugt folgende Anforderungen zu stellen: Kompakte Bauweise: Einbau in Innenvolumen einer Küvette mit Abmaßen 10 mm x 10 mm x 45 mm, geringstmögliche Störung des Weges/Durchtritts zur optischen Vermessung des Gerinnungsverlaufes; chemische Beständigkeit: Wechselwirkungen, wie Oxidation und Reduktion, zwischen Elektrodenwerkstoff und Blutplasma bzw. CaCl₂ müssen vermieden werden; Ladungsneutralität: ausschließlich elektrische Effekte aufgrund der Gerinnung sollen gemessen werden; Form- und Lagestabilität: Lage und Position der Elektroden sollen während des Versuches unverändert bleiben; Ausgestaltung der Elektroden: Plattenkondensatoranordnung, bei horizontaler Anordnung muss Durchtritt der wandernden Gerinnungsfront (von oben nach unten) gewährleistet sein; Elektrodenmaterial: gute elektrische Leitfähigkeit zur Vermessung von Spannungen bis zum µV-Bereich; Wiederverwendbarkeit: zur Durchführung von weiteren Messungen, insbesondere Vergleichsmessungen, muss Elektrodenanordnung leicht zu reinigen sein.

Als Elektrodenmaterial wird bevorzugt ein Edelmetall, bevorzugt Gold, gewählt. Für die Herstellung der Elektrodenanordnungen wird Gold in Form von feinem Blech, Draht und Netz verwendet. Mehrere Goldteile können mittels Punktschweißens miteinander verbunden werden, beispielsweise ein Draht an ein Blech angepunktet. Als besonders vorteilhaft hat sich die Ausbildung eines Rahmens einstückig mit einer Zuleitung zum Aufspannen eines Goldnetzes erwiesen. Das Goldnetz wird durch Umlage von freien Randabschnitten am Rahmen befestigt und zu einer Fläche aufgespannt. Weiter können metallische Implantate, wie Stents, als Ganzes oder abschnittsweise als Elektrodenteile eingesetzt werden. Hierbei können Stentausschnitte entsprechend angeordnet werden. Bei der Verwendung von typischerweise rohrzylinderförmigen Stents wird eine Zylinderkondensatoranordnung aus zwei Stents unterschiedlicher Größe oder einem Stent mit einer mittig angeordneten Stiftelektrode oder einem Draht ausgebildet.

Als Trägermaterial zur räumlichen Anordnung der Elektroden werden thermoplastische Kunststoffe, wie Abschnitte einer Hart-PVC-Platte oder eines Plexiglasrohres, eingesetzt. Derartige Kunststoffe sind in der Medizintechnik weit verbreitet, beispielsweise zum Bau von Schläuchen, Spritzen und Endoskopen. Die 1,1mm dicken Plattenabschnitte werden zu einem Trägerteil verbunden, dessen Außenabmaße entsprechend dem Innenvolumen der Küvette gewählt sind. Anschließend werden die Gold-Elektroden entsprechend der gewünschten Anordnung, hier eine Plattenkondensatoranordnung, befestigt. Hierbei findet Kunststoffuniversalkleber vorzugsweise mit Dosierhilfe Verwendung. Die freien Zuleitungen zu den die Kondensatorplatten bildenden Elektroden werden durch Ummantelung mit einem PE-Schlauch oder einem Schrumpfschlauch elektrisch isoliert.

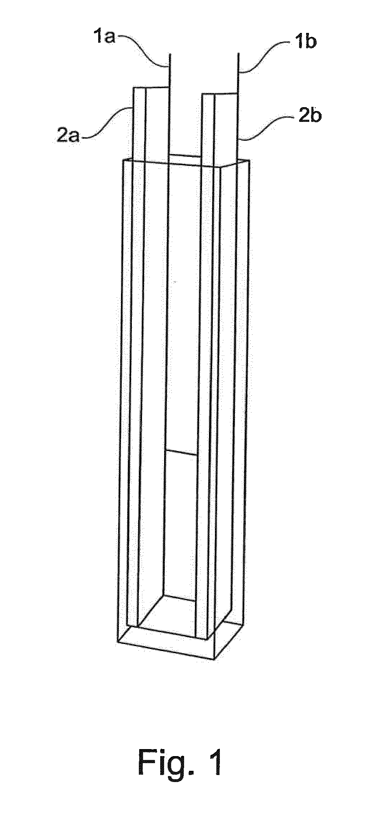

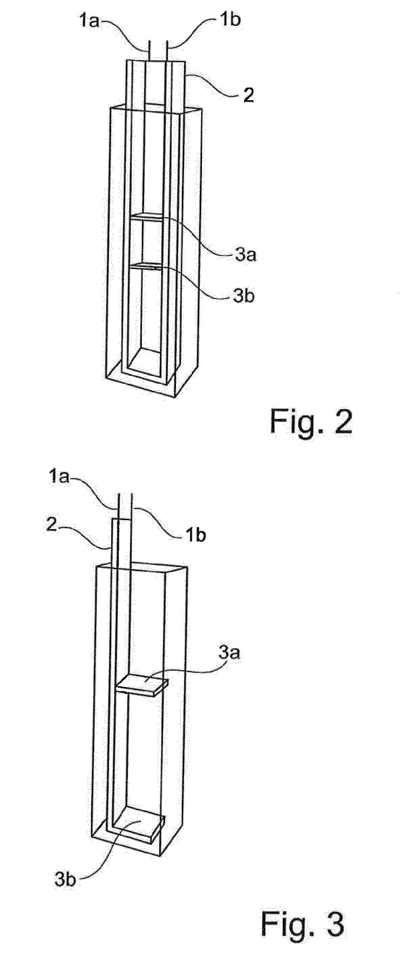

Als horizontale Plattenkondensatoranordnungen sind die in den Figuren 1-3 in perspektivischer Darstellung gezeigten Elektrodenmodelle realisiert worden. Bei dem in Figur 1 gezeigten Elektrodenmodell werden zwei abgewinkelte Golddrähte (1a, 1b) auf zwei getrennte Platten (2a, 2b) unter Freigabe von freien Golddrahtenden befestigt. Die im Elektrodenmodell gemäß Figur 1 in den sichtbaren Bereich ragenden Golddrahtenden erlauben eine einfache optische Bestimmung der Lage der Elektroden und des Zeitpunktes der Passierung der Gerinnungsfront an der (oberen bzw. unteren) Elektrode. Bei den in den Figuren 2-3 gezeigten Elektromodellen wird Golddraht (1a, 1b) mit einem Durchmesser von 0,5 mm verwendet, welcher die nötige Biegefestigkeit zur Ausbildung von formstabilen Ebenen aufweist. Der Golddraht ist an einer Platte (2) befestigt. Durch eine mäanderförmige Ausbildung und Anordnung von mehreren Windungen bzw. Schleifen wird eine Kondensatorplatte bzw. -ebene (3a, 3b) aus einem Drahtstück geformt, wobei die Windungen bzw. Schleifen zur Vermeidung von möglichen Einflüssen von PVC auf den Spannungsverlauf von den Platten leicht beabstandet werden.

Die horizontalen Kondensatorplatten bzw. -ebenen können von Calciumchloridlösung bzw. von der Gerinnungsfront durchschritten werden. Zwischen den Platten bzw. Ebenen des Kondensators kann sich ein homogenes elektrisches Feld aufbauen und dieses vermessen werden. Bei den in den Figuren 1-2 gezeigten Elektrodenmodellen beträgt der vertikale Abstand der Elektroden zum Küvettenboden 25 mm für die obere und 18 mm für die untere Elektrode, bei dem in Figur 3 gezeigten Elektrodenmodell 25 mm für die obere und 0 mm für die untere Elektrode. Die Abstände können anforderungsabhängig gewählt werden und variieren während einer Messreihe typischerweise zwischen 0 mm und 25 mm. In Elektrodenmodell gemäß Figur 3 kann der gesamte Gerinnungsverlauf elektrisch vermessen werden, da die Lage der unteren Elektroden der maximalen Endlage der Gerinnungsfront entspricht.

Es versteht sich, dass mehrere Elektroden in einer Küvette angeordnet werden können. In einer derartigen Mehrfachanordnung können über unterschiedlich ausgestaltete Elektrodenanordnungen Messwerte erfasst und/oder das Blutsystem mit einer elektrischen Größe beaufschlagt werden. Bei Plexiglasrohrabschnitten zur räumlichen Anordnung der Elektroden kann eine gesonderte Befestigung der Elektroden entfallen und die gewünschte Anordnung wird lose durch Einsetzen entsprechender Rohrabschnitte und Elektrodenteile in die Küvette erreicht. Weiter ergibt sich der Vorteil, dass unterschiedliche Elektrodenanordnungen in einfacher Weise zusammengesetzt und zu Umbau- und/oder Reinigungszwecke auseinandergebaut werden können.

Die Elektrodenanordnungen werden in eine Küvette eingesetzt, welche in die Küvettenhalterung eingesetzt wird, und werden in dieser Position über Klemmen an das Elektrometer angeschlossen. Die Küvette wird mit Blutplasma derart befüllt, dass beide Elektroden nahezu vollständig eingetaucht sind oder bei einer horizontalen Plattenkondensatoranordnung die untere Elektrode in das Blutplasma eingetaucht ist und die obere Elektrode die oberseitige Grenzfläche des Blutplasmas darstellt. Die Aktivierung der Gerinnung erfolgt anschließend mit Zugabe der CaCl₂-Lösung im Verhältnis 1:4, so dass das Blutplasma ausgehend von der Grenzfläche unten verlaufend gerinnt. Die Calciumchloridlösung liegt in der Konzentration 0,025 mol/l vor.

Inhibiertes Blutplasma, genauer Citratplasma, wird zweckmäßigerweise tiefgefroren bevorratet. Nach dem Auftauen eines beispielsweise 5 ml beinhaltenden Röhrchens wird eine Menge von ca. 2,5 ml Blutplasma in eine Küvette eingefüllt. Die Küvette wird in senkrechter Anordnung in die Küvettenhalterung eingesetzt, anschließend wird die Elektrodenanordnung eingesetzt. Nach Zugabe von CaCl₂-Lösung dringt ausgehend von der Grenzfläche die CaCl₂-Lösung in das Blutplasma ein und aktiviert dessen Gerinnung. Bei der Gerinnung wird das gelbe, durchsichtige Blutplasma weiß und opak.

Über das Programm wird die gewünschte Aufzeichnung eingestellt und gestartet. Neben den Bildern werden die über die Elektrodenanordnung gemessene Spannung und die im Innern der Verdunklungskiste gemessene Temperatur zeitsynchronisiert aufgezeichnet. Die Dauer eines Messablaufs (Zeitfenster) beträgt typischerweise 24, 48 oder 72 Stunden, wobei eine Bild- und Messwerteaufzeichnung pro Minute stattfindet. Der Datensatz wird zur Dokumentation und weiteren Auswertung abgespeichert. Die elektrischen Messungen werden gleichzeitig mit einer optischen Vermessung des Gerinnungsverlaufs durchgeführt. Zur Anwendung des erfindungsgemäßen Verfahrens ist es jedoch ausreichend, elektrische Messungen durchzuführen, um den Fortschritt der Gerinnung, anders ausgedrückt den Gerinnungsverlauf, zu beobachten.

Bei der Untersuchung von Blut wird dieses bei der Abnahme inhibiert, anders ausgedrückt Citratblut hergestellt, und innerhalb von 48 Stunden nach der Abnahme auf elektrische Effekte während der Gerinnung untersucht, wobei eine Zugabe von CaCl₂-Lösung zum Blut im Verhältnis 1:4 erfolgt. Ausgehend von der Grenzfläche dringt die CaCl₂-Lösung in das Blut ein und aktiviert dessen Gerinnung. Hierbei kann zumindest im sichtbaren Wellenlängenbereich keine optische Messung durchgeführt werden, es sind jedoch optische Messungen in geeigneten Wellenlängenbereichen und/oder rheologische Messungen denkbar.

Um sicherzustellen, dass über die jeweilige Elektrodenanordnung lediglich aufgrund der Gerinnung des Blutplasmas auftretende elektrische Effekte gemessen werden, wird in Vergleichsversuchen das Blutplasma durch Ringer-Lösung ersetzt, welche mit CaCl₂-Lösung "aktiviert" wird. Ringer-Lösung ist eine wässrige Infusionslösung.

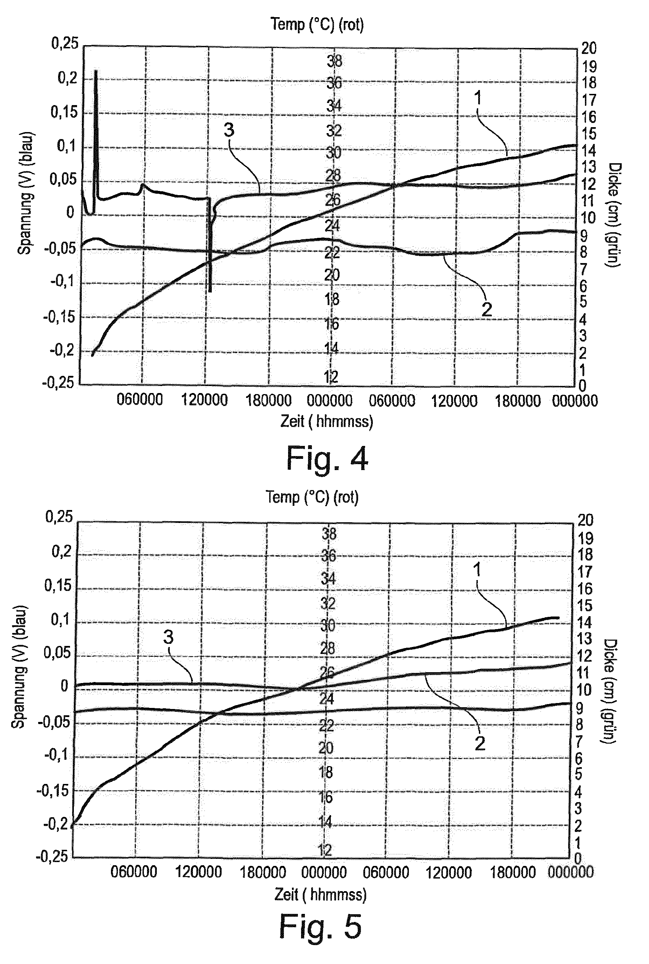

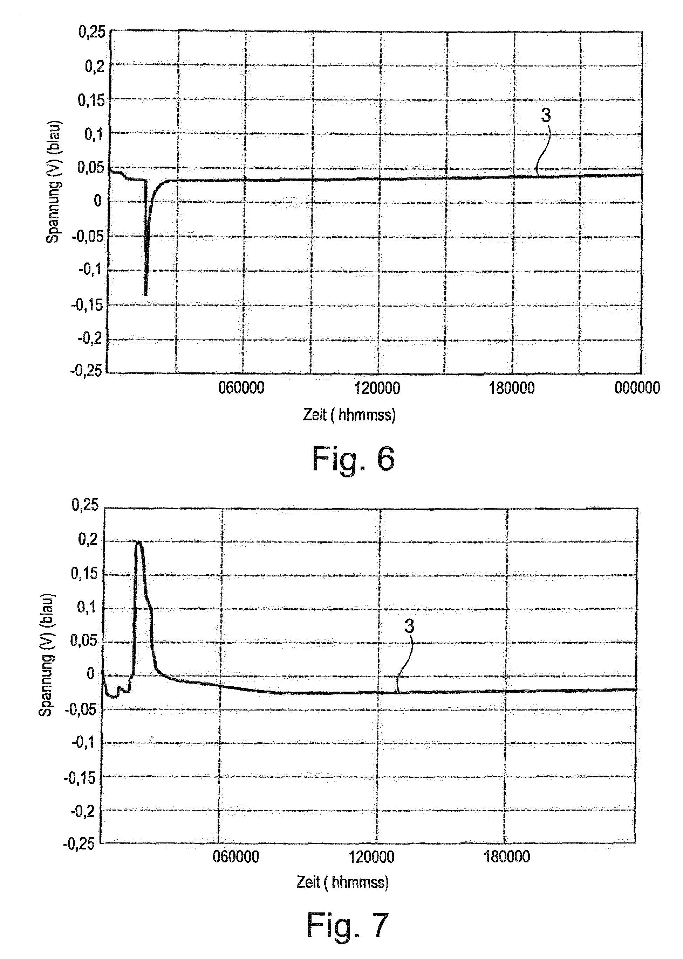

In den Figuren 4-7 ist für den jeweils bestimmten Versuch der zeitliche Verlauf der Dicke der Gerinnungsschicht in mm (Kurve 1), der Temperatur in °C (Kurve 2) und der Spannung in V (Kurve 3) gezeigt. Figur 4 zeigt die Ergebnisse (48 Stunden) für die in Figur 1 gezeigte Elektrodenanordnung. Figur 5 zeigt die Ergebnisse (48 Stunden) für die in Figur 2 gezeigte Elektrodenanordnung. Figur 6 zeigt die Ergebnisse (24 Stunden) für die in Figur 3 gezeigte Elektrodenanordnung. Figur 7 zeigt die Ergebnisse (24 Stunden) für eine vergleichbare Elektrodenanordnung Mit den Pfeilen sind die Positionen markiert, an denen die Gerinnungsfront eine Elektrode passiert, was mit einem Peak in der Spannungskurve korreliert. Die Ergebnisse der Messungen an Blutplasma zeigen, dass Spannungswerte im Bereich ± 150mV bis zu ± 1V messbar sind, dass die größten Spannungswerte bei Elektrodenmodellen mit horizontaler Kondensationsanordnung erreicht werden, und dass der Durchtritt der Gerinnungsfront durch die (obere bzw. untere) Elektrode in Spannungskurven erkennbar ist. Der Verlauf der Spannung ist vergleichbar mit den für Kunststoffsysteme gemessenen Verläufen.

Die Ergebnisse der Messungen an Blut zeigen, dass Spannungswerte im Bereich ± 200mV messbar sind, dass der Durchtritt der Gerinnungsfront durch Elektroden in Spannungskurven als Peak erkennbar ist. Beide Elektroden sind in zunächst inhibiertes Blut eingetaucht. Mit Ringer-Lösung durchgeführte Vergleichsversuche zeigen, dass die gemessenen elektrischen Werte, hier Spannungswerte, auf die Gerinnung des Blutes zurückzuführen sind.

## Patentansprüche

1. Eine zumindest teilweise in einem Blutsystem anordenbare Vorrichtung zur Detektion, Vermessung und/oder Beeinflussung der Gerinnung von Blutsystemen, umfassend:
- eine erste Elektrode,
- eine zweite Elektrode,
wobei die Elektroden zumindest teilweise in das Blutsystem eintauchbar sind und mit einem Messgerät zur Erfassung mindestens einer elektrischen Messgröße und optional mit einer Spannungs- und/oder Stromquelle elektrisch leitend verbindbar sind.

2. Die Vorrichtung gemäß Anspruch 1, wobei die Elektroden aus einem Metall, bevorzugt einem Edelmetall, besonders bevorzugt Gold, gefertigt sind; und/oder die Elektroden als Plattenelektroden, zylinderförmige Elektroden, Drahtelektroden und/oder Stiftelektroden ausgebildet sind.

3. Implantierbares System, insbesondere Stentanordnung, mit einer Vorrichtung nach Anspruch 1 oder 2.

4. Das System nach Anspruch 3, wobei die erste Elektrode als ein erster rohrförmiger Körper mit einem Innendurchmesser D1 und einer Wandung, die ein Innenvolumen V1 umschließt, ausgebildet ist.

5. Das System nach Anspruch 3 oder 4, wobei die zweite Elektrode als ein zweiter rohrförmiger Körper mit einem Außendurchmesser D2, wobei D2<D1, und/oder als eine stift- oder drahtförmige Komponente ausgebildet ist.

6. Das System nach Anspruch 5, wobei der zweite rohrförmige Körper und/oder die stift-oder drahtförmige Komponente in dem Innenvolumen V1 angebracht und somit zumindest teilweise von der Wandung des ersten rohrförmigen Körpers umschlossen ist/sind.

7. Das System nach Anspruch 5 oder 6, wobei der zweite rohrförmige Körper und/oder die stift- oder drahtförmige Komponente und der erste rohrförmige Körper koaxial zueinander angeordnet sind.

8. Eine Anordnung mit der Vorrichtung oder dem System nach einem der vorstehenden Ansprüche, und einem Computer, der programmtechnisch so eingerichtet ist, dass bei dem Überschreiten eines kritischen Messwertes der elektrischen Messgröße ein äußeres elektrisches Gegenfeld durch die Strom- bzw. Spannungsquelle generiert wird und bevorzugt beim Unterschreiten des kritischen Grenzwertes wieder deaktiviert wird.

9. Ein Gerät zur Blutbehandlung und/oder Blutaufbewahrung außerhalb des menschlichen Körpers, enthaltend die Vorrichtung nach Anspruch 1 oder 2 oder die Anordnung nach Anspruch 8 mit Anspruch 1 oder 2, wobei es sich bei dem Gerät bevorzugt um ein Dialysegerät oder ein Blutinfusionsgerät handelt.

10. Ein Verfahren zur Detektion, Vermessung und/oder Beeinflussung der Gerinnung von Blutsystemen außerhalb des menschlichen Körpers, umfassend:
- die Bereitstellung einer Vorrichtung, insbesondere nach Anspruch 1 oder 2, die zumindest eine erste Elektrode und eine zweite Elektrode umfasst, wobei die Elektroden mit einem Messgerät zur Erfassung mindestens einer elektrischen Messgröße und optional mit einer Spannungs- und/oder Stromquelle elektrisch leitend verbunden sind,
- in Kontakt bringen der Elektroden mit einem Blutsystem, wie Blutplasma oder Blut, wobei die Elektroden zumindest teilweise in das Blutsystem eingetaucht sind und kein äußeres elektrisches Feld an die Elektroden angelegt wird,
- Messung der zumindest einen elektrischen Messgröße.

11. Das Verfahrens gemäß Anspruch 10, wobei das Verfahren unter Verwendung der Vorrichtung nach Anspruch 1 oder 2, der Anordnung nach Anspruch 8 mit Anspruch 1 oder 2, und/oder dem Gerät nach Anspruch 9 durchgeführt wird.

12. Das Verfahren nach Anspruch 10 oder 11, wobei ein kritischer Grenzwert der elektrischen Messgröße festgelegt wird, die gemessenen Werte der elektrischen Messgröße mit dem festgelegten kritischen Grenzwert abgeglichen werden, und beim Überschreiten des kritischen Grenzwertes die Generierung eines äußeren elektrischen Gegenfeldes, das gegenüber dem durch die elektrische Messgröße generierten elektrischen Feld entgegengesetzt ausgerichtet ist, durch die Spannungs- oder Stromquelle erfolgt, und beim Unterschreiten des kritischen Grenzwertes das äußere elektrische Gegenfeld bevorzugt wieder deaktiviert wird.
